(19) Europäisches Patentamt
European Patent Office
Office européen des brevets

(11) Veröffentlichungsnummer: **0 254 865 B1**

(12) **EUROPÄISCHE PATENTSCHRIFT**

(45) Veröffentlichungstag der Patentschrift: **02.01.91 Patentblatt 91/01**

(51) Int. Cl.⁵: C07C 69/92, C09K 19/20, C11D 1/74

(21) Anmeldenummer: **87108944.7**

(22) Anmeldetag: **23.06.87**

(54) **Amphiphile 4-alkoxypolyethoxybenzoesäureester, ihre Herstellung und Verwendung.**

(30) Priorität: **02.07.86 DE 3622086**

(43) Veröffentlichungstag der Anmeldung: **03.02.88 Patentblatt 88/05**

(45) Bekanntmachung des Hinweises auf die Patenterteilung: **02.01.91 Patentblatt 91/01**

(84) Benannte Vertragsstaaten: **AT BE CH DE ES FR GB IT LI NL SE**

(56) Entgegenhaltungen:
**US-A- 4 519 936**
**MOLECULAR CRYSTALS AND LIQUID CRYSTALS, Band 114, Nr. 1/3, 1984, Seiten 237-247, London, GB; C. DECOBERT et al.: "Synthesis and mesomorphysm of some new ferro-electric smectic liquid crystals"**
**JOURNAL OF THE CHEMICAL SOCIETY, FARADAY TRANSACTIONS I, Band 79, Nr. 1, 1983, Seiten 975-1000, London, GB; D.J. MITCHELL et al.: "Phase behaviour of polyoxyethylene surfactants with water"**

(73) Patentinhaber: **BASF Aktiengesellschaft**
**Carl-Bosch-Strasse 38**
**D-6700 Ludwigshafen (DE)**

(72) Erfinder: **Kock, Hans-Jakob, Dr.**
**Benckisserstrasse 63**
**D-6700 Ludwigshafen (DE)**
Erfinder: **Winkler, Ekhard, Dr.**
**Pfalzring 74**
**D-6704 Mutterstadt (DE)**
Erfinder: **Baur, Richard, Dr.**
**Nelkenstrasse 1**
**D-6704 Mutterstadt (DE)**
Erfinder: **Finkelmann, Heino, Prof. Dr.**
**Fillibachstrasse 31**
**D-7800 Freiburg i. Br. (DE)**
Erfinder: **Schaftheutle, Markus**
**Hofackerstrasse 96-98**
**D-7800 Freiburg i. Br. (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

**Beschreibung**

Amphiphile Verbindungen, die aus hydrophilen und hydrophoben Einheiten aufgebaut sind, bilden in wäßrigen Systemen oberhalb der kristischen Mizellkonzentration mizellare Strukturen aus, die bei geeigneter Struktur und in bestimmten Konzentrationsbereichen als flüssigkristalline Phasen (Mesophasen) vorliegen. Diese Mesophasen können aus sphärischen, stäbchenförmigen bzw. scheibchenförmigen Mizellen aufgebaut sein, wobei in einem binären System auch verschiedene Mesophasen existieren können. Die Strukturen der Mesophasen können über Texturuntersuchungen im Polarisationsmikroskop bestimmt werden. Eine ausführliche Sammlung von Textur-Struktur-Zuordnungen ist in Textures of Liquid Crystals, Ed. Demus, Richter Verlag Chemie (1978) zu finden. Aus der Literatur (Mitchell et al, J. Chem. Soc. Farad. Trans. I. 89, 975 (1983) und Lang et al, J.Chem. Phys., 73, 5849 (1980)) ist beispielsweise das Phasenverhalten und insbesondere die Existenz von mesomorphen Phasen in Abhängigkeit von der Balance zwischen hydrophilen und hydrophoben Einheiten von nichtionischen Tensiden des Aufbaus $CH_3\text{-}(CH_2)_y\text{-}(O\text{-}CH_2\text{-}CH_2)_x\text{-}OH$bekannt. Durch geeignete Wahl der Relation von x zu y lassen sich binäre System aus Tensid und Wasser realisieren, die in einem bestimmten Temperaturbereich Mesophasen aufweisen.

H. Finkelmann et al, Colloid & Polymer Sci. 260, 56-65 (1982) berichten über eingehende vergleichende Untersuchungen von monomeren Amphiphilen und deren polymerisierten Analoga. Aufgrund der verbesserten Löslichkeit der amphiphilen Seitenkettenpolymeren wird die Mischungslücke im binären System aus Tensid und Wasser zu höheren Temperaturen verschoben.

Durch den Einbau starrer hydrophober Einheiten in amphiphile Verbindungen konnten Lühmann et al (vgl. Makromol. Chem. 186, 1059 (1985) und Colloid & Polym. Sci. 264, 189 (1986)) zeigen, daß derartige Verbindungen im binären System mit Wasser scheibchenförmige (diskotische) Mizellen ausbilden und daß eine einer flüssigkristallinen lamellaren $L_\alpha$-Phase vorgelagerte lyotrope nematische Phase existiert.

Die bislang bekannten mesomorphen Tensidsysteme haben den Nachteil, daß sie eine Vielzahl von flüssigkristallinen Phasen in eng begrenzten Konzentrationsbereichen besitzen und insbesondere die durch ihre ausgezeichneten Fließeigenschaften charakterisierten nematischen Phasen nur in äußerst engen Konzentrations- und Temperaturbereichen als homogene Phasen existieren. Smektische Phasen mit $S_c$-Struktur sind bisher nicht bekannt.

Der Erfindung liegt die Aufgabe zugrunde, nichtionische amphiphile Verbindungen zur Verfügung zu stellen, die in wäßriger Lösung plattenförmige (diskotische) Mizellen ausbilden können und die mindestens eine smektische Phase aufweisen.

Die Aufgabe wird erfindungsgemäß gelöst mit nichtionischen 4-Alkoxypolyethoxybenzoesäureestern der Formel

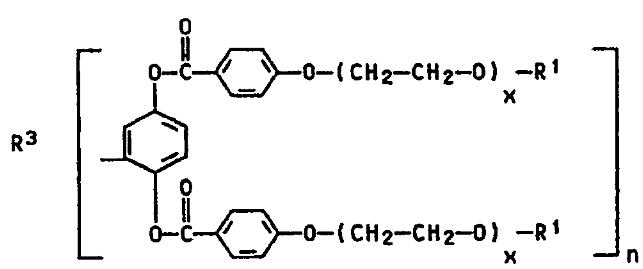

in der $R^3$ einen n-wertigen Rest der Formel

H- , 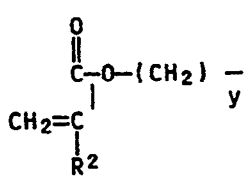 oder 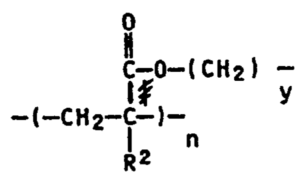

n = 1 oder eine ganze Zahl über 1,
$R^1$ = $C_1$-bis $C_4$-Alkyl,
$R^2$ = -H oder $-CH_3$,
x = 3 bis 12 und

y = 3 bis 15
bedeuten.

Erfindungsgemäße Stoffe, die als Rest $R^3$ denjenigen der Formel

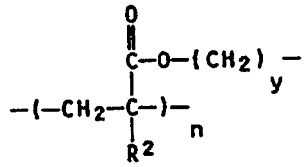

enthalten, sind polymere Verbindungen. Ihre Formel wird hier vereinfacht in der allgemein gebräuchlichen offenen Form wiedergegeben, obwohl diese wie die überwiegende Zahl polymerer Stoffe an beiden Seiten der Polymerkette Endgruppen tragen, wie folgende schematische Formel zeigt :

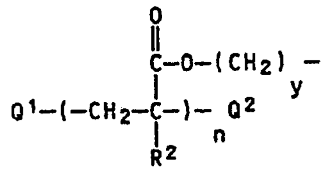

Dabei stellen $Q^1$ und $Q^2$ die Endgruppen dar, deren Natur bekanntlich von den jeweiligen Polymerisationsbedingungen abhängt und in aller Regel nicht bekannt ist, da sie nur durch sehr aufwendige Untersuchungen feststellbar ist und zumeist für das Verhalten eines polymeren Stoffes keine wesentliche Bedeutung hat. Zur Problematik der Endgruppen s. z.B. Houben-Weyl, "Methoden der organischen Chemie", 4. Auflage, Band 14/1, Seiten 116 bis 118.

Bei den polymeren erfindungsgemäßen Stoffen ist n mindestens 2 und vorzugsweise bis zu 400 ; besonders vorteilhaft sind diejenigen polymeren erfindungsgemäßen Stoffe, in denen n eine ganze Zahl 20 bis 200 ist.

Die nicht polymeren Stoffe gemäß der Erfindung entsprechen der Formel

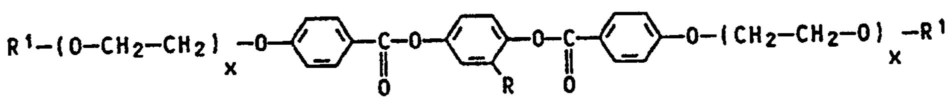 (I),

in der R = H, 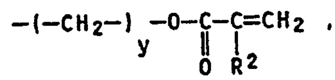,

$R^1$ = $C_1$- bis $C_4$-Alkyl
$R^2$ = H, $CH_3$,
x = 3 bis 12 und
y = 3 bis 15
bedeuten. Bei x kann es sich auch um einen mittleren Ethoxylierungsgrad handeln, d.h. es können Mischungen von Verbindungen der Formel I vorliegen, die einen unterschiedlichen Ethoxylierungsgrad haben, wobei der mittlere Wert von x 3 bis 12 beträgt.

In den Verbindungen der Formel I stellen die beiden Gruppierungen

$$R^1-(O-CH_2-CH_2)_x-$$

zwei hydrophile Flügelgruppen dar, während der verbleibende Teil des Moleküls als hydrophober starrer Kern aufzufassen ist. Stoffe der Formel

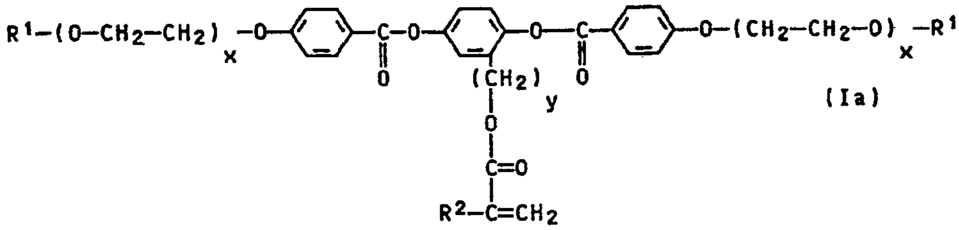

(Ia)

in der
$R^1$ = $C_1$- bis $C_4$-Alkyl, vorzugsweise $CH_3$,
$R^2$ = H, $CH_3$,
x = 3 bis 12, vorzugsweise 5 bis 8 und
y = 3 bis 15, vorzugsweise 8 bis 12
bedeuten,
können in Gegenwart von Radikale bildenden Initiatoren und gegebenenfalls Reglern polymerisiert werden. Man erhält dabei die oben besprochenen polymeren Stoffe, bei denen Reste der Formel I mit R = H in Form von Seitenketten so an eine Polymerkette fixiert sind, daß die molekulare Längsachse der hydrophoben Einheit der Verbindung der Formel I quer zur verknüpfenden Alkylenkette liegt. Überraschenderweise wurde gefunden, daß die Verbindungen der Formel I, die aus einem starren hydrophoben Kern und zwei hydrophilen Flügelgruppen aufgebaut sind, im binären System mit Wasser nur eine definierte Mizellgeometrie ausbilden, nämlich scheibchenförmige (diskotische) Mizellen, deren Mizellgröße (Dicke) gezielt durch die Wahl der hydrophoben Einheit eingestellt werden kann. Diese Mizellen bilden in bestimmten Konzentrationsbereichen alleine lyotrop smektische Phasen aus. Auch für die polymeren nichtionischen Stoffe, die durch Polymerisation der Verbindungen der Formel Ia erhältlich sind, gilt, daß sie im binären System aus Tensid und Wasser flüssigkristalline Phasen aufweisen.

Die Verbindungen der Formel I werden z.B. in einer mehrstufigen Umsetzung erhalten, bei der man zunächst eine Verbindung der Formel

$$R^1-(O-CH_2-CH_2)_x-Hal \qquad (II),$$

in der
$R^1$ = $C_1$- bis $C_4$-Alkyl, vorzugsweise $CH_3$, x = 1 bis 12 und
Hal = Cl, Br und J
bedeuten, mit 4-Hydroxybenzoesäure-$C_1$- bis $C_4$- bis $C_4$-Alkylester unter Halogenabspaltung in Gegenwart von Basen zu Verbindungen der Formel

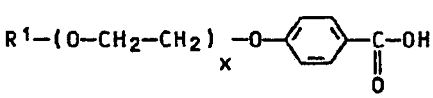

(III)

umsetzt.

Geeignete 4-Hydroxybenzoesäureester sind beispielsweise der Methylester, Ethylester, Isopropylester, n-Propylester, Isobutylester und n-Butylester der 4-Hydroxybenzoesäure. Vorzugsweise setzt man 4-Hydroxybenzoesäuremethylester ein. Als Basen kommen vor allem Natronlauge, Kalilauge oder Alkalialkoholate in Betracht. Die Umsetzung kann in Substanz oder in einem Lösemittel, wie Dioxan, Wasser, Methanol oder Dimethylsulfoxid durchgeführt werden. Die Verbindungen der Formel III werden in das entsprechende Säurechlorid oder Säurebromid der Formel

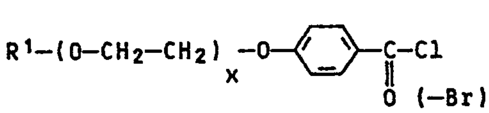

(IV),

überführt, das dann durch Umsetzung mit Verbindungen der Formel

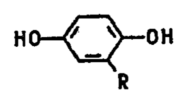 (V),

in der
R = H oder

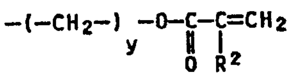

$R^2$ = H, $CH_3$, vorzugsweise $CH_3$ und
y = 3 bis 15, vorzugsweise 8 bis 12,
bedeutet, zur Reaktion gebracht wird.

Die Umsetzung wird in Gegenwart von tertiären Aminen, z.B. Pyridin, Triethylamin oder Tributylamin durchgeführt. Die Umsetzung erfolgt vorzugsweise in einem Lösemittel, wie Dioxan, Tetrahydrofuran oder Dichlormethan. Man erhält Lösungen von nichtionischen Tensiden der Formel I, aus denen die Verbindungen der Formel I durch Abdampfen des Lösemittels isoliert werden können. Gegebenenfalls wird eine Reinigung vorgeschaltet, z.B. Chromatographie an Kieselgel.

Man kann aber z.B. auch in an sich bekannter Weise zunächst 4-Hydroxybenzoesäure oder ihre Ester, ihr Chlorid oder ihr Bromid mit der halben molaren Menge einer Verbindung der Formel V zu einem Ester der Formel

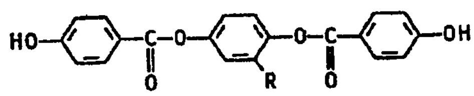

umsetzen und diese dann in üblicher Weise an den endständigen Hydroxylgruppen mit Ethylenoxid in solcher Menge umsetzen, daß der mittlere Ethoxylierungsgrad jeder Polyglykolkette den Wert x hat, und das so erhaltene Ethoxylierungsprodukt dann in an sich bekannter Weise mit einem den Rest $R^1$ einführenden Agens, z.B. einem Alkylchlorid $R^1$-Cl oder mit Dimethylsulfat, zu einem erfindungsgemäßen Stoff umsetzen.

Ebenso ist es z.B. möglich, zunächst einen 4-Hydroxybenzoesäure-$C_1$- bis $C_4$-alkylester mit Ethylenoxid zu einem Ethoxylierungsprodukt der Formel

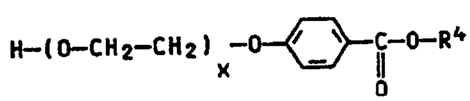 (IIIa)

in der $R^4$ den $C_1$- bis $C_4$-Alkylrest bedeutet, umzusetzen, in dieses Produkt dann, wie oben angegeben, den Rest $R^1$ einzufügen, wobei ein Produkt der Formel

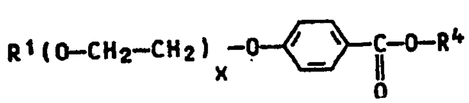 (IIIb)

entsteht, und dieses dann durch Umesterung mit einer Verbindung der Formel V zu einem erfindungsgemäßen Stoff umzuwandeln.

Die Verbindungen der Formel Ia enthalten eine ethylenisch ungesättigte Doppelbindung und können daher der Polymerisation unterworfen werden. Die Polymerisation wird mit Hilfe von Initiatoren gestartet, die unter den Polymerisationsbedingungen in Radikale zerfallen. Geeignete Initiatoren sind die üblicherweise bei Polymerisationen verwendeten Peroxide, Hydroperoxide, Wasserstoffperoxid, und Azoverbindungen, wie Azobisisobuttersäuredinitril und insbesondere wasserlösliche Azoverbindungen, z.B. 2,2'-Azobis-(2,4-dimethylvaleronitril), 2,2'-(N,N'-Dimethylisobutyr amidin), 4,4-Azobis(4-cyanpentancarbonsäure) und 2,2'-Azobis-(2,4-dimethylvaleronitril). Als Polymerisationsinitiatoren eignen sich auch die bei Polymerisationen üblicherweise in Betracht kommenden Redoxinitiatoren. Die Initiatoren werden in den üblichen Mengen eingesetzt, z.B. in einer Menge von 0,05 bis 1,0 Gew.%, bezogen auf die Verbindung der

Formel Ia. Die Polymerisation wird vorzugsweise in dem Temperaturbereich von 0 bis 60°C durchgeführt. Es können dabei auch in üblicher Weise Regler, wie Dodecylmercaptan, Mercaptoethanol, Thioessigsäure oder Hydroxylammoniumsulfat, mitverwendet werden. Die Polymerisation kann prinzipiell nach den bekannten Verfahren der Lösungspolymerisation, umgekehrten Suspensionspolymerisation und Wasser-in-Öl-Polymerisation durchgeführt werden. Die Polymerisate können nach Entfernen des Lösungsmittels in reiner Form isoliert werden. Der Polymerisationsgrad der Polymerisate liegt üblicherweise in dem Bereich von 2 bis 400, vorzugsweise von 20 bis 200 (bestimmt mit Hilfe der Gelpermeationschromatographie an Standard-Polystyrol mit Tetrahydrofuran). Die Verbindungen der Formel I und die Polymeren, die durch Polymerisieren von Verbindungen der Formel Ia erhältlich sind, sind nichtionische Substanzen, die in wäßriger Lösung scheibchenförmige (diskotische) Mizellen ausbilden und die mindestens eine smektische Phase aufweisen. Sowohl die Monomeren als auch die Polymeren können in Wasch- oder Reinigungsmittelformulierungen zur Verstärkung der Waschkraft eingesetzt werden. Die Mengen, die dafür in Betracht kommen, liegen in dem Bereich von 0,1 bis 10 Gew.%, bezogen auf die Wasch- oder Reinigungsmittelformulierung. Sie können weiterhin als oberflächenaktive Zusätze für die verschiedensten Tensid-Anwendungen eingesetzt werden, beispielsweise in galvanischen Bädern, bei der Herstellung von Leiterplatten für die Elektronik und in Pesticid-Formulierungen. Außerdem sind sie vorteilhaft als Flüssigkristalle in den verschiedensten optischen, elektronischen und optoelektronischen Anwendungen einsetzbar.

Die in den Beispielen angegebenen Teile sind Gewichtsteile, die Angaben in Prozent beziehen sich auf das Gewicht der Stoffe.

Beispiel 1

Herstellung des Benzoesäureesters der Formel

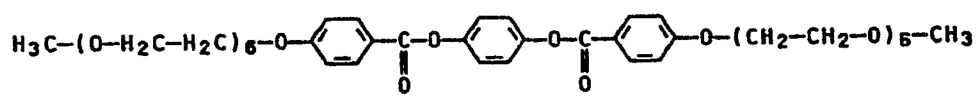 (6)

a) 101,16 g (≈ 0,6 mol) 3,6,9-Trioxadecanol werden mit 44,6 ml (≈ 0,6 mol) Thionylchlorid und 51,7 ml (≈ 0,64 mol) Pyridin in einer Tetrahydrofuran-Lösung durch Erhitzen unter Rückfluß (12 h) umgesetzt zu 3,6,9-Trioxadecylchlorid

$$H_3C-[OCH_2CH_2]_3-Cl \quad (1)$$

Nach Abkühlung der Mischung wird mehrmals mit Methylethylketon (MEK) extrahiert und die MEK-Phase anschließend mit $NaHCO_3$ neutralisiert und über $Na_2SO_4$ getrocknet. Nach Entfernen des MEK wird der Rückstand bei 30 Pa und 30-37°C destilliert. Ausbeute : 90 %.

b) Zunächst werden 160 g (≈ 1,05 mol) 3,6-Dioxaoctan-1,8-diol mit 11,6 g (≈ 0,5 mol) Natrium bei einer Temperatur von 110°C innerhalb von 12 Stunden zum Monoalkoholat umgesetzt. Während der Reaktionszeit wird die Mischung intensiv gerührt. Danach gibt man Kaliumjodid als Katalysator zu und fügt innerhalb von 4 bis 5 Stunden 91,9 g (≈ 0,5 mol) der Verbindung (1) tropfenweise zu der auf 110° C gehaltenen Reaktionsmischung zu. Nach vollständiger Zugabe der Verbindung (1) wird das Reaktionsgemisch noch 64 Stunden bei einer Temperatur von 160° C gerührt. Das Reaktionsgemisch wird dann auf Raumtemperatur abgekühlt, filtriert und fraktioniert. In der Fraktion bei 60 Pa und 150 bis 160° C destilliert die Verbindung der Formel

$$H_3C-[O-CH_2-CH_2]_6-OH \quad (2)$$

d.h. 3,6,9,12,15,18-Hexaoxanonadecanol ab.

c) 39 g (≈ 0,143 mol) der Verbindung (2) werden analog zur Synthese der Verbindung (1) mit 10,1 ml (≈ 0,139 mol) Thionylchlorid in 11,5 ml (≈ 0,143 mol) Pyridin zur Verbindung der Formel

$$H_3C-[O-CH_2-CH_2]_6-Cl \qquad (3)$$

chloriert.

d) Eine Lösung von 8,05g (≈ 0,143 mol) Kaliumhydroxid in 55 ml Wasser wird mit 21,8 g 4-Hydroxybenzoesäuremethylester und 6 ml (≈ 0,148 mol) Methanol versetzt. Die Lösung wird anschließend unter Rühren zu einer Mischung von 45 g (≈ 0,143 mol) der Verbindung der Formel (3) und einer katalytischen Menge von Kaliumjodid gegeben und 12 Stunden unter Rückfluß gehalten. Nach einiger Zeit tritt eine Phasen separation ein. Um die Reaktion zu Ende zu führen, setzt man zum Reaktionsgemisch 11,5 g KOH zu und erhitzt die Mischung 4 Tage lang zum Sieden unter Rückfluß. Hierbei verschwindet die Phasen separation. Durch Zugabe von Salzsäure wird das Reaktionsgemisch auf einen pH-Wert von 1 angesäuert und mehrmals mit MEK extrahiert. Das MEK wird anschließend aus dem Extrakt abdestilliert. Es verbleibt ein öliger Rückstand, der zweimal in Diethylether bei -16° C umkristallisiert und über Phosphorpentoxid im Vakuum getrocknet wird. Man erhält einen leicht gelblichen Stoff der Formel

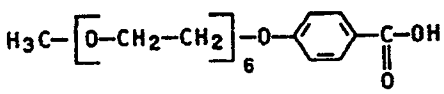

(4)

in einer Ausbeute von 40,5 g = 65,5 %. Der Schmelzpunkt beträgt 38 bis 39° C.

e) 3 g (≈ 0,007 mol) der Verbindung (4) werden in 5 ml (≈ 0,07 mol) Thionylchlorid gelöst und 5 Stunden mit Dimethylformamid als Katalysator zum Sieden unter Rückfluß erhitzt. Danach wird das restliche Thionylchlorid abdestilliert und der Rückstand bei 100 Pa und einer Temperatur von 225 bis 226° C destilliert. Man erhält das Säurechlorid der Formel

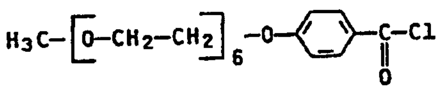

(5)

in einer Ausbeute von 2,07 g = 65,5%.

f) Zu einer Lösung von 258,8 mg (≈ 0,0024 mol) Hydrochinon und 0,66 ml (≈ 0,0048 mol) Triethylamin in 5 ml wasserfreiem Methylenchlorid gibt man 2,05 g (≈ 0,0045 mol) der Verbindung der Formel (5) gelöst in 1 ml wasserfreiem Methylenchlorid tropfenweise bei einer Temperatur von -20° C zu. Die Mischung wird 2 Tage bei Raumtemperatur gerührt. Dann entfernt man das Methylenchlorid und chromatographiert den Rückstand an handelsüblichem Kieselgel mit einer Korngröße von 60 bis 63 µm mit einer Mischung aus Ether und Aceton im Verhältnis 1 : 2. Man erhält ein Festprodukt der Formel (6), das einen Schmelzpunkt von 19,8° C hat. Die Ausbeute beträgt 75 %.

In binären Systemen Benzoesäureester (6)/$H_2O$ bilden sich in verdünnter Lösung Mizellen, wobei die c.m.c. (kritische Mizellbildungskonzentration) über Oberflächenspannungsmessungen ermittelt wird (Tabelle 1).

Tabelle 1

c.m.c. des Tensids (6) in $H_2O$

| | c.m.c. (Gew.%) |
|---|---|
| 10° C | $8{,}15 \cdot 10^{-2}$ |
| 20° C | $6{,}46 \cdot 10^{-2}$ |
| 30° C | $5{,}63 \cdot 10^{-2}$ |

Untersuchung des lyotropen Phasenverhaltens

Zur Untersuchung des lyotropen Phasenverhaltens von Amphiphilen eignet sich in besonderem Maße das Polarisationsmikroskop mit einer Temperiereinrichtung für die Probe, da hier gleichzeitig Phasenübergänge bestimmt und optisch verfolgt werden können. Zu Voruntersuchungen und zur Klärung, ob bzw. welche Mesophasen bei einem Tensid-Wasser-System auftreten, wird die Kontaktpräparation verwendet.

a) Kontaktpräparate

Hierzu wird jeweils eine kleine Menge der zu untersuchenden Verbindung auf einen Objektträger gegeben und ein Deckgläschen so darüber gelegt, daß es mit einer Kante auf einem seitlich der Probe liegenden zweiten Deckgläschen aufliegt. Zwischen das schief liegende Deckgläschen und dem Objektträger läßt man einen Tropfen Wasser einziehen. Das Wasser fließt um die amphiphile Verbindung und mit der Zeit bildet sich durch Diffusion ein Konzentrationsgradient vom reinen Wasser zum Amphiphil aus. Das Präparat wird in einen Heiztisch geschoben, der sich im Strahlengang eines Polarisationsmikroskops befindet. Die Phasenumwandlung können nun durch Aufheizen ober Abkühlen im Mikroskop beobachtet werden, wobei die Heizrate nicht größer als 1°C/min gewählt werden sollte.

b) Aufnahme eines Phasendiagramms durch definierte Mischungen

Zur Aufnahme eines Phasendiagramms werden verschiedene Tensid-Wasser-Mischungen angesetzt und in Abhängigkeit von der Temperatur untersucht.

Da die Probe aufgrund ihrer geringen Viskosität leicht zwischen Objektträger und Deckgläschen auseinander fließt, sollt durch Distanzhalter (Spacer) zwischen ihnen für eine ausreichend dicke Probenschicht gesorgt werden. Zur Präparation werden 50-mg-Proben verschiedener Zusammensetzung in Gewichtsprozent an Tensid und Wasser auf der Analysenwaage in kleine zylindrische Teflonkammern eingewogen. Nach Hinzugeben einer Edelstahl kugel wird mit einer Schwingmühle eine homogene Mischung hergestellt. Einen Teil der Mischung gibt man anschließend auf einen Objektträger, auf den man zuvor kleine Stückchen einer hitzebeständigen Folie (z.B. handelsübliche Backfolie) als Spacer gelegt hat. Nach Auflegen eines Deckgläschens wird die Probe, um einem Wasserverlust beim Aufheizen vorzubeugen, mit einem lösungsmittelfreien Epoxidharz eingeklebt. Die Untersuchung erfolgt wiederum im Heiztisch mit einem Polarisationsmikroskop.

Das binäre System aus dem Benzoesäureester (6) und Wasser zeigt im Bereich von 53 bis 84 Gew.% Benzoesäureester (6) smektische Phasen, die über einem Temperaturbereich von 8,7°C bis 33,5°C existieren. Die homogenen smektischen Phasen existieren im Bereich von 54-75 Gew.% Benzoesäureester (6) und zwischen 8,7°C und 28°C. Von besonderer Bedeurung ist dabei, daß die bei hohen Temperaturen ausgebildete Phase eine smektische $S_c$-Phase ist. Diese Phase ist bisher für lyotrope Flüssigkristalle unbekannt.

Beispiel 2

Herstellung des Benzoesäureesters der Formel

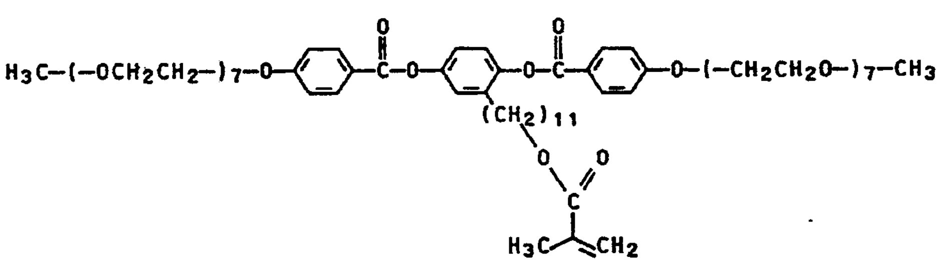

(7)

Analog dem in Beispiel 1 beschriebenen Verfahren wird aus Tetraethylenglykol mit $SOCl_2$ das Tetraethylenglykoldichlorid

$$Cl-[CH_2CH_2O]_3-CH_2CH_2-Cl$$ (8)

hergestellt, das dann mit dem Mono-Natriumsalz des Triethylenglykolmonomethylethers zu der Verbindung der Formel

$$H_3C-[O-CH_2CH_2]_7-Cl$$ (9)

umgesetzt wird. Durch Reaktion von (9) mit 4-Hydroxybenzoesäuremethylester resultiert die Verbindung der Formel

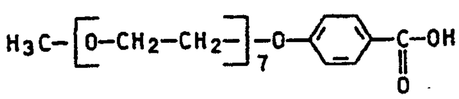

(10),

die mit Thionylchlorid in das entsprechende Säurechlorid der Formel

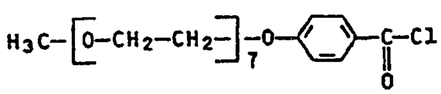

(11)

überführt wird.

Für die Synthese von (7) wird dann nach F. Hessel und H. Finkelmann, Polym. Bull. 14, 375 (1985), Chinon über das Bromwasserstoffaddukt des Undecensäuremethylesters zum entsprechenden Hydrochinonderivat der Formel

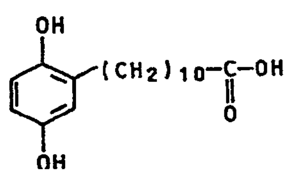

(12)

umgesetzt, das mit $LiAlH_4$ zum Alkoholderivat

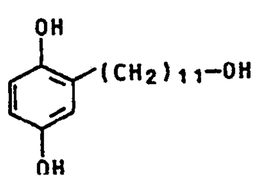

(13)

reduziert und danach mit Methacrylsäure zu der Verbindung der Formel

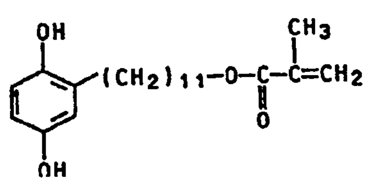

(14)

verestert wird. Die Umsetzung von (11) mit (14) ergibt den Benzoesäureester der oben angegebenen Formel (7). Hierzu werden 3 mol (11) mit 1 mol (14) und 3 mol Pyridin in Dichlormethan bei Temperaturen unterhalb 0° C umgestzt. Nach säulenchromatographischer Reinigung erhält man den Benzoesäureester der Formel (7) mit einer Ausbeute von 75 %.

Im binären System aus Benzoesäureester (7) und Wasser bildet sich in einem breiten Konzentrationsbereich eine homogene smektische Phase aus. Bei niedrigen Tensidkonzentrationen in wäßriger Lösung koexistiert oberhalb der Schmelztemperatur des Wassers eine smektische Phase mit einer hochverdünnten isotropen mizellaren Lösung. Bei hohen Tensidkonzentrationen koexistiert die smektische Phase mit einer konzentrierten isotropen mizellaren Tensidlösung. Unterhalb 0° C koexistiert Eis mit der smektischen Phase.

Beispiel 3

Das im Beispiel 2 beschriebene Benzoesäureester (7) wird in Tetrahydrofuran mit einer Feststoffkonzentration von 23 % gelöst und mit 0,19 Mol.% Azobisisobuttersäuredinitril, bezogen auf Benzoesäureester (7), versetzt. Das Reaktionsgemisch wird gerührt, auf eine Temperatur von 60° C erwärmt und 18 Stunden bei dieser Temperatur gehalten. Danach wird das Polymerisat mit Hexan gefällt und anschließend im Vakuum getrocknet.

Das Kontaktpräparat zeigt eine lamellare smektische Phase mit einem Existenzbereich von -12,9°C bis 41,8°C.

## Ansprüche

1. 4-Alkoxypolyethoxybenzoesäureester der Formel

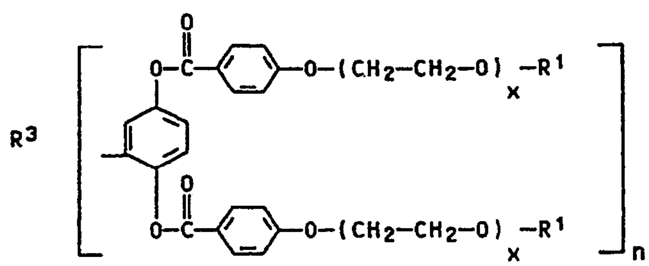

in der $R^3$ einen n-wertigen Rest der Formel

H- , 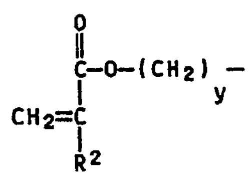 oder 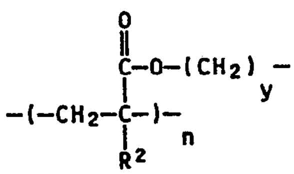

n = 1 oder eine ganze Zahl über 1,
$R^1$ = $C_1$- bis $C_4$-Alkyl,
$R^2$ = -H oder -$CH_3$,
x = 3 bis 12 und
y = 3 bis 15
bedeuten.

2. Stoffe nach Anspruch 1, in denen n eine ganze Zahl von 2 bis 400 ist.

3. Stoffe nach Anspruch 1, in denen n eine ganze Zahl von 20 bis 200 ist.

4. Stoffe nach Anspruch 1, gekennzeichnet durch die Formel I

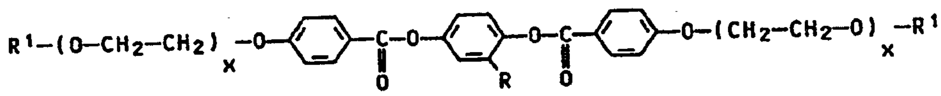 (I),

in der R = H, 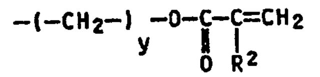

$R^1$ = $C_1$- bis $C_4$-Alkyl,
$R^2$ = H, $CH_3$,
x = 3 bis 12 und
y = 3 bis 15
bedeuten.

5. Stoffe nach Anspruch 1, gekennzeichnet durch die Formel Ia

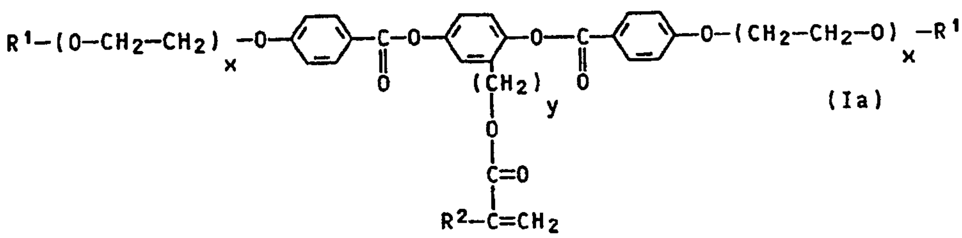

(Ia)

in der
$R^1$ = $C_1$- bis $C_4$-Alkyl
$R^2$ = H, $CH_3$
x = 3 bis 12 und
y = 3 bis 15
bedeuten.

6. Verfahren zur Herstellung der 4-Alkoxypolyethoxybenzoesäureester gemäß den Ansprüchen 4 und 5, dadurch gekennzeichnet, daß man eine Verbindung der Formel

$$R^1-(O-CH_2-CH_2)_x-Hal \qquad (II)$$

in der $R^1$ = $C_1$- bis $C_4$-Alkyl,- x = 3 bis 12 und
Hal = Cl, Br und J
bedeuten, mit 4-Hydroxybenzoesäure-$C_1$- bis $C_4$-Alkylester unter Halogenabspaltung in Gegenwart von Basen zu Verbindungen der Formel

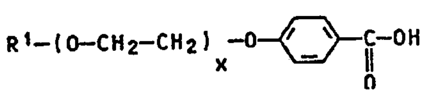 (III)

umsetzt, die Verbindungen der Formel III in das entsprechende Säurechlorid oder Säurebromid

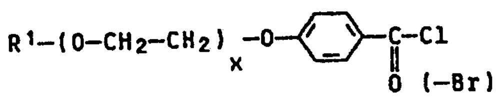 (IV).

überführt, und dieses dann mit Verbindungen der Formel

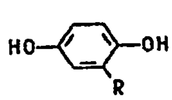 (V).

in der R = H,

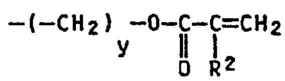

$R^2$ = H, $CH_3$ und
y = 3 bis 15
bedeuten, umsetzt.

7. Verfahren zur Herstellung von polymeren 4-Alkoxypolyethoxybenzoesäureestern gemäß den Ansprüchen 2 und 3, dadurch gekennzeichnet, daß man Stoffe gemäß Anspruch 5 in Gegenwart von Radikale bildenden Polymerisationsinitiatoren polymerisiert.

8. Polymere 4-Alkoxypolyethoxybenzoesäureester erhalten nach dem Verfahren gemäß Anspruch 7.

9. Verwendung der Stoffe gemäß den Ansprüchen 1 bis 5 und 8 als Tenside.

10. Verwendung der Stoffe gemäß den Ansprüchen 1 bis 5 und 8 als Zusatz zu Wasch- und Reinigungsmitteln in einer Menge von 0,1 bis 10 Gew.%, bezogen auf die Wasch- und Reinigungsmittelformulierung.

11. Verwendung der Stoffe gemäß den Ansprüchen 1 bis 5 und 8 als Flüssigkristalle in Optik, Elektronik und Optoelektronik.

## Claims

1. A 4-alkoxypolyethoxybenzoate of the formula

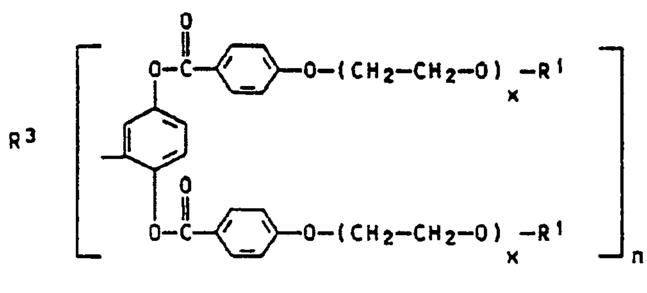

where $R^3$ is an n-valent radical of the formula

H-

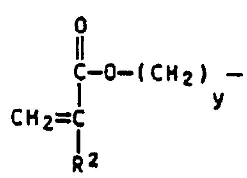

or

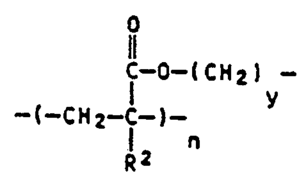

n is 1 or an integer greater than 1, $R^1$ is $C_1$-$C_4$,-alkyl, $R^2$ is -H or -$CH_3$, x is from 3 to 12 and y is from 3 to 15.

2. A substance as claimed in claim 1, wherein n is an integer from 2 to 400.

3. A substance as claimed in claim 1, wherein n is an integer from 20 to 200.

4. A substance as claimed in claim 1, of the formula I

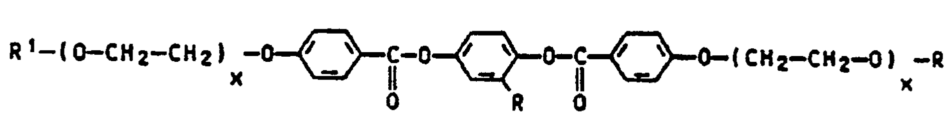

(I)

where R is H or

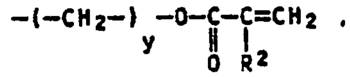

$R^1$ is $C_1$-$C_4$,-alkyl, $R^2$ is H or $CH_3$, x is from 3 to 12 and y is from 3 to 15.

5. A substance as claimed in claim 1, of the formula Ia

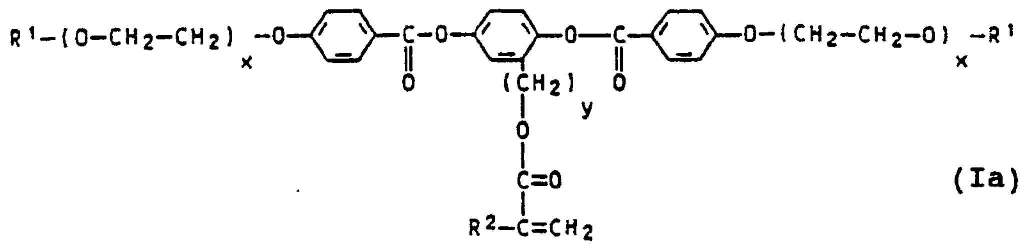

(Ia)

where $R^1$ is $C_1$-$C_4$,-alkyl, $R^2$ is H or $CH_3$, x is from 3 to 12 and y is from 3 to 15.

6. A process for the preparation of a 4-alkoxypolyethoxybenzoate as claimed in claims 4 and 5, wherein a compound of the formula

$$R^1-(O-CH_2-CH_2)_x-Hal \qquad (II)$$

where $R^1$ is $C_1$-$C_4$,-alkyl, x is from 3 to 12 and Hal is C1, Br or I, is reacted with a $C_1$-$C_4$,-alkyl 4-hydroxybenzoate with elimination of halogen in the presence of a base to give a compound of the formula

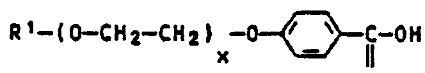

(III)

0 the compound of the formula 111 is converted to the corresponding acyl chloride or acyl bromide

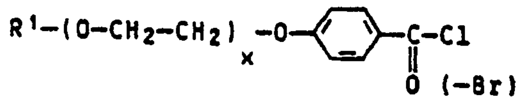

(IV)

and the latter is then reacted with a compound of the fo

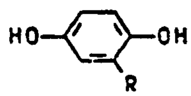

(V)

where R is H or

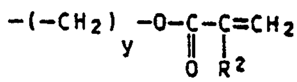

$R^2$ is H or $CH_3$ and y is from 3 to 15.

7. A process for the preparation of a polymeric 4-alkoxypolyethoxybenzoate as claimed in claims 2 and 3, wherein a substance as claimed in claim 5 is polymerized in the presence of a free radical polymerization initiator.

8. A polymeric 4-alkoxypolyethoxybenzoate obtained by a process as claimed in claim 7.

9. Use of a substance as claimed in claims 1 to 5 and 8 as a surfactant.

10. Use of a substance as claimed in claims 1 to 5 and 8 as an additive to detergents and cleaning agents in an amount of from 0.1 to 10% by weight, based on the detergent or cleaning agent formulation.

11. Use of a substance as claimed in claims 1 to 5 and 8 as liquid crystals for optical, electronic and opto-electronic applications.

## Revendications

1. Esters d'acides 4-alcoxypolyéthoxybenzoïques répondant à la formule

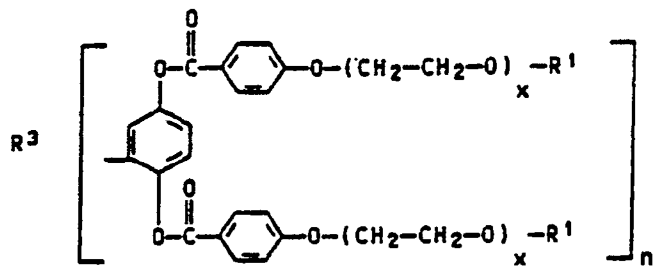

dans laquelle $R^3$ représente un radical n-valent répondant à la formule

H- , 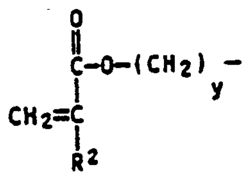 ou 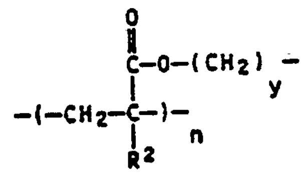

n est égal à 1 ou représente un nombre entier supérieur à 1,
$R^1$ représente un radical alkyle en $C_1$ à $C_4$,
$R^2$ représente -H ou $-CH_3$,
x a une valeur qui varie de 3 à 12 et
y a une valeur qui varie de 3 à 15.

2. Substances suivant la revendication 1, dans lesquelles n représente un nombre entier dont la valeur varie de 2 à 400.

3. Substances suivant la revendication 1, dans lesquelles n représente un nombre entier dont la valeur varie de 20 à 200.

4. Substances suivant la revendication 1, caractérisées en ce qu'elles répondent à la formule 1

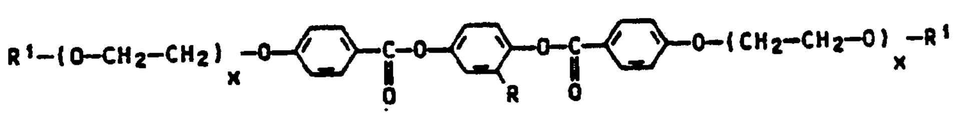 (I)

où R = H, 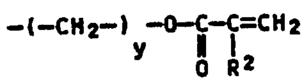

$R^1$ représente un radical alkyle en $C_1$ à $C_4$,
$R^2$ représente un atome d'hydrogène, le radical $CH_3$,
x a une valeur qui varie de 3 à 12 et
y a une valeur qui varie de 3 à 15.

5. Substances suivant la revendication 1, caractérisées en ce qu'elles repondent à la formule la

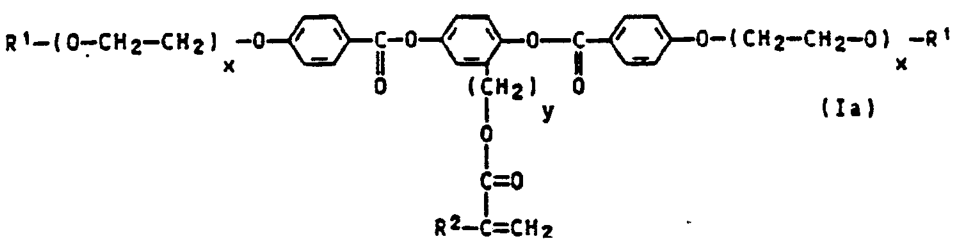

(Ia)

dans laquelle
$R^1$ représente un radical alkyle en $C_1$ à $C_4$,
$R^2$ représente un atome d'hydrogène, le radical $CH_3$,
x a une valeur qui varie de 3 à 12 et
y a une valeur qui varie de 3 à 15.

6. Procédé de préparation d'esters d'acides 4-alcoxypolyéthoxybenzoïques suivant les revendications 4 et 5, caractérisé en ce que l'on convertit un composé répondant à la formule

$$R^1-(O-CH_2-CH_2)_x-Hal \qquad (II)$$

dans laquelle $R^1$ represente un radical alkyle en $C_1$ à $C_4$, x a une valeur qui varie de 3 à 12 et Hal represente un atome de chlore, de brome ou d'iode, à l'aide d'esters alkyliques en $C_1$ à $C_4$ de l'acide 4-hydroxybenzoïque, avec séparation d'halogène et en présence de bases, en composés de la formule

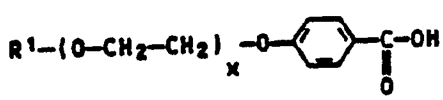

(III)

on convertit les composés de la formule III en le chlorure d'acide ou le bromure d'acide correspondant

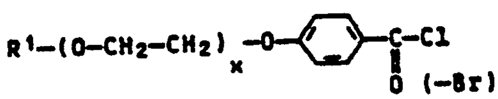

(IV).

et on fait ensuite réagir celui-ci avec des composés de la formule

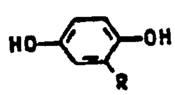

(V)

dans laquelle R = H,

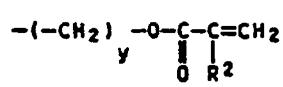

$R^2$ = H, $CH_3$ et y a une valeur qui varie de 3 à 15.

7. Procédé de préparation d'esters d'acides 4alcoxypolyéthoxybenzolques suivant les revendications 2 et 3, caractérisé en ce que l'on polymérise des substances selon la revendication 5, en présence d'amorceurs de polymérisation qui engendrent des radicaux.

8. Esters d'acides 4-alcoxypolyéthoxybenzoïques polymères, obtenus par mise en oeuvre du procédé selon la revendication 7.

9. Utilisation des substances suivant les revendications 1 à 5 et 8, à titre d'agents tensioactifs ou surfactifs.

10. Utilisation des substances suivant les revendications 1 à 5 et 8, à titre d'additifs pour des produits détergents et des produits de lavage ou de lessivage, en une proportion de 0,1 à 10% en poids par rapport à la composition des produits détergents ou de lavage ou de lessivage.

11. Utilisation des substances suivant es revendications 1 à 5 et 8, à titre de cristaux liquides dans le domaine optique, électronique et optoélectronique.